# EUROPÄISCHE PATENTSCHRIFT

(11) **EP 1 487 332 B1**
(45) Veröffentlichungstag und Bekanntmachung des Hinweises auf die Patenterteilung: **27.06.2012**
(21) Anmeldenummer: 03744728.1
(22) Anmeldetag: 13.03.2003
(51) Int. Cl.: A61B 5/04, A61B 5/0424

(54) **Messvorrichtung zur Reduzierung von Messfehlern**
Measuring device for reducing measuring errors
Dispositif de mesure pour reduire les erreurs de mesure

(30) Priorität: 25.03.2002 AT 4592002
(43) Veröffentlichungstag der Anmeldung: 22.12.2004
(73) Patentinhaber: Stockinger, Christian, 3512 Mautern/Do. (AT)
(72) Erfinder: Stockinger, Christian, 3512 Mautern/Do. (AT)
(74) Vertreter: Calderbank, Thomas Roger
(86) Internationale Anmeldenummer: PCT/AT2003/000073
(87) Internationale Veröffentlichungsnummer: WO 2003/079896

(56) Entgegenhaltungen:
- EP-A2- 1 080 683
- WO-A-00/78209
- US-B1- 6 314 315
- PATENT ABSTRACTS OF JAPAN vol. 018, no. 454 (P-1791), 24. August 1994 (1994-08-24) -& JP 06 148358 A (CASIO COMPUT CO LTD), 27. Mai 1994 (1994-05-27)

## Beschreibung

### 1.1. Einleitung

Die vorliegende Erfindung bezieht sich darauf, Messgrößen möglichst fehlerfrei von einer interessierenden Messstelle abzunehmen.

Als Beispiel seien physiologische Messgrößen vom menschlichen oder tierischen Körper angeführt (etwa Hautwiderstand, Temperatur, Durchblutung etc.). Die Sensoren für diese Messgrößen können auf Gegenständen wie z.B. einer Lenk- oder Steuereinrichtung, einem Dateneingabegerät, einem mobilen Datenverarbeitungsgerät oder einem mobilen Telefon angebracht sein, das der Anwender im Moment der Messung verwendet.

Dies ist für die verschiedensten Anwendungen wie z.B. Zustandsüberwachung, Stressüberwachung, Monitoring, Entspannungstraining, Fitnesstraining, Spiel, Leistungssteigerung, Diagnose und Training von Körperfunktionen zur Heilung und Linderung von Beschwerden und Krankheiten, arbeitsergonomische Anwendungen etc. verwendbar.

Exemplarisch werden in diesem Text die Messgrößen Hautwiderstand und die Temperatur zur Beschreibung herangezogen.

Prinzipiell können auch andere Messgrößen wie Durchblutung, Sauerstoffsättigung, Oberflächenhärte, elektrische Aktivität, Wärmeabgabe etc. sowie Messgrößen von anderen Oberflächen als menschlichen oder tierischen Körperoberflächen mit dieser Vorrichtung unter Verminderung der Messartefakte gemessen werden.

Die so erfassten Messgrößen und die Hilfsgrößen werden gemessen und in Daten verarbeitenden Geräten weiter verarbeitet.

### 1.2. Stand der Technik

### 1.2.1. Entgegenhaltungen

Wie im Patent anmeldung JP111118636A (Tokai Rika Denki KK), 30. April 1999 (30.04.99) gezeigt, werden bereits eine Mehrzahl von Sensorelementen in einem Sensor verwendet, die gemeinsam betrieben werden. Hier werden Sensoren des gleichen Typs verwendet, die in Beziehung zueinander stehen und deren Signale gemeinsam ausgewertet und daraus Ausgangsgrößen berechnet werden, die aus einem Sensor alleine u.U. nicht ermittelt werden können. Die anmeldung beschreibt eine Methode, ein Sensorarray gut geschützt und miniaturisiert herzustellen.

Das Patent JP9215667A (Nippon Koden Corp.), 19. August 1997 (19.08.97) beschreibt eine Mehrzahl von Sensoren einer Messanordnung, die gemeinsam ein Gesamtergebnis liefern. Für den gezeigten EKG-Sensor arbeiten drei Arten von Sensoren zusammen (invertierender Kontakt, nicht invertierender Kontakt und Referenzkontakt eines Instrumtenverstäkers), um ein möglichst fehlerfreies Signal von der Haut abzuleiten. Der gemeinsame Aufbau in ein Gerät ermöglicht Handlichkeit und eine Verminderung von Messfehlern. Durch die federnde Anordnung der Kontakte wird eine Anpassung an die Hautoberfläche ermöglicht.

Die Neuartigkeit der vorliegenden Erfindung gegenüber den beiden Entgegenhaltungen besteht darin, dass zwei Arten von Sensoren oder zwei Arten von Information, die aus Sensoren abgeleitet werden kann in einem gemeinsamen Sensorelement verwendet werden. Dabei wird die eine Sensorinformation und die bekannte geometrische Lage der Sensoren zueinander für die Beurteilung der Korrektheit der anderen Sensorinformation herangezogen (ob und welche Sensorelemente in Kontakt, ob und welche Sensorelemente vollständig abgedeckt).

Um Messfehler noch weiter zu vermindern sind zusätzlich die Sensorelemente beweglich oder formbar ausgeführt und damit bei Bewegungen der Messstelle nachführbar sowie die Kontaktflächen oder der gesamte Sensor für die Messung des Hautwiderstandes zur Erzielung eines vorteilhaften möglichst gleichbleibenden Auflagedruckes federnd angeordnet.

### 1.2.2. Weitere Veröffentlichungen

In DE 199 59 576 A1 wird zwar die Erfassung von physiologischen Parametern unter schwierigen Bedingungen beschrieben, jedoch nicht mit besonderen Sicherungsmechanismen für die Gewährleistung der Berührung, der Abdeckung und vollständigen Abdeckung, des nicht verlorenen Kontaktes und im Falle des Hautwiderstandes des konstanten Anpressdruckes im Rahmen einer solchen Anordnung.

Beim Beispiel der physiologischen Messungen auf der Haut werden in EP1 109 382 A2 zwar Sicherungsmechanismen für die Gewährleistung der Berührung durch die Haut mit zwei unterschiedlichen Sensorsystemen beschrieben, jedoch keine für die Abdeckung und vollständigen Abdeckung, des nicht verlorenen Kontaktes und im Falle des Hautwiderstandes des konstanten Anpressdruckes im Rahmen einer solchen Anordnung.

Wie z.B. in US 6067 468 dargelegt wird das Anbringen von Sensoren oft einfach vorausgesetzt. Die Anwender werden üblicherweise bestenfalls darauf hingewiesen, auf die korrekte und solide Anbringung von Sensorelementen zu achten und möglichst wenige oder keine Bewegungen während der Messung zu machen.

JP 6-148358 offenbart ein elektronisches Instrument für die Durchführung von Messungen an einem menschlichen Finger, mit einer Elektrode in einer für die Aufnahme eines Fingers geeigneten Vertiefung um somit einen stabilen Kontakt zwischen Finger und Elektrode sicherzustellen.

WO 00/78209 offenbart ein System zur Ermittlung einer ungeeigneten Anlegung einer Puls-Oximeter-Sonde an die Haut eines Patienten.

### Zusammenfassung der Erfindung

Die gegenständliche Erfindung beinhaltet eine Messvorrichtung zum Messen von physiologischen Größen von einer menschlichen oder tierischen Hautoberfläche und zum Ermitteln von ungenauem Anlegen von Messstellen auf Messsensoren und zur Anwendung in robusten Meßsystemen, die Werte unter realistischen und schwierigen Bedingungen messen. Die Messvorrichtung besteht aus: einer Mehrzahl von Sensorelementen zur Durchführung von physiologischen Messungen, wobei die Sensorelemente auf einer Sensorfläche befestigt sind und in eine Anzahl von Hauptsensoren und Hilfssensoren aufgeteilt sind; und einer Daten-Verarbeitungs-Einheit zum Unterscheiden zwischen Signalen von den Hauptsensoren und den Hilfssensoren zur Ermittlung von ungenauem Anlegen von Messstellen auf Messsensoren. Die relativen Positionen der Haupt- und Hilfssensoren zueinander sind der Daten-Verarbeitungs-Einheit bekannt, so daß durch Signale der Hauptsensoren und Hilfssensoren eine Kontaktfläche feststellbar ist, innerhalb der alle Hauptsensoren vollständig abgedeckt sind. Die Daten-Verarbeitungs-Einheit ist so ausgelegt daß sie nur Signale von vollständig bedeckten Hauptsensoren verwendet und, falls erwünscht, für vollständig bedeckte Hilfssensoren innerhalb der ermittelten Kontaktfläche für tatsächliche Messungen.

### 1.4. Beschreibung der Erfindung

### 1.4.1. Detektion des Sensorelementkontaktes sowie der vollständigen Abdeckung der Sensorelemente

### 1.4.1.1. Mittels Hauptsensorelementen und Hilfssensorelementen

Die Erfindung ist durch folgendes gekennzeichnet: Die auf dem Sensor angebrachten Sensorelenlente sind in Hauptsensoren und Hilfssensoren getrennt. Es gibt eine Anzahl von Haupt- und Hilfssensoren auf der die Messstelle berührenden Sensorfläche. Die geometrische Lage der Haupt- und Hilfssensorelemente zueinander ist dem Messsystein bekannt. Falls erforderlich können eine oder mehrere gemeinsame Referenzsensoren verwendet werden

Wird nun detektiert, welche Hilfs-Sensoren von der Messstelle berührt werden, kann das Messsystem auf die Kontaktfläche rückschließen. Alle innerhalb dieser ermittelten Kontaktfläche liegenden Haupt-Sensoren sind nun vollständig abgedeckt und ihre Signale werden für die aktuelle Messung herangezogen. Dabei, und das ist sehr vorteilhaft für die Funktion, müssen die Hilfs-Sensoren nicht vollständig abgedeckt sein, das heißt, sie müssen nur ungefähre Messdaten liefern wie Kontakt oder nicht Kontakt.

Die Unterscheidung zwischen den Signalen der Haupt-Sensorelemente und den Signalen der Hilfs-Sensorelemente für das Datenverarbeitungssystem muss gewährleistet sein.

Alternativ können die Messungen zur Detektion des Sensorelementkontaktes und die Haupt-Messung mit der gleichen Messgröße und auch den gleichen Messsensoren zeitlich hintereinander durchgeführt werden. Dabei wird angenommen dass während den Hauptmessungen (zwischen den Detektionsmessungen) der Sensorkontakt mit der Messstelle unverändert bleibt.

### 1.4.1.1.1. Beispiel Daumensensor für Computermaus

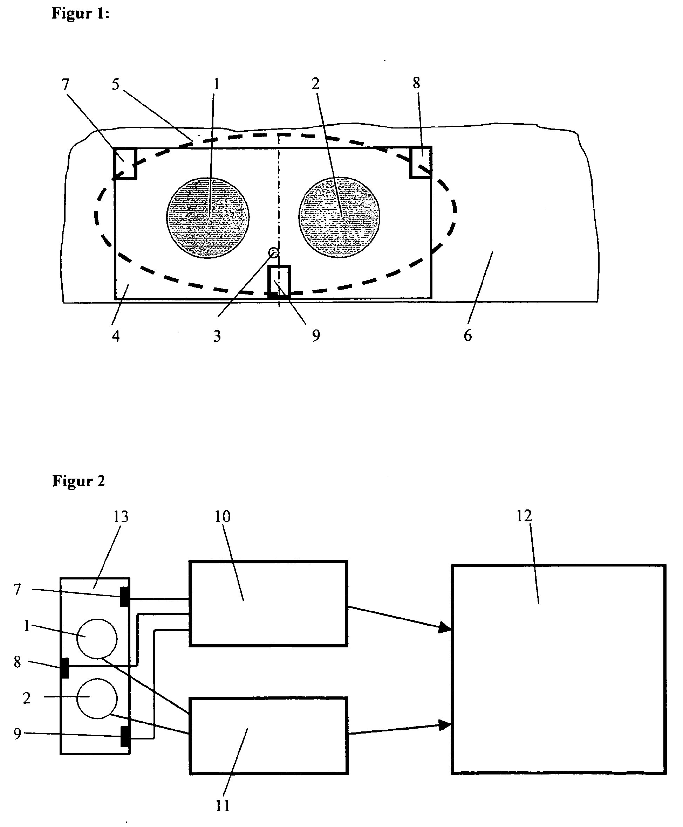

Als Beispiel sei ein Daumensensor für eine Computermaus nach Figur 1 "Schematische Ansicht der Detektion der vollständigen Abdeckung für einen Daumensensor einer Computermaus" angeführt.

Um zu gewährleisten dass alle Haupt-Sensorelemente, (1) (2) für den Hautwiderstand, (3) für die Temperatur, erstens abgedeckt und zweitens vollständig abgedeckt sind und so ihre Funktion möglichst fehlerfrei erfüllen können, ist dieser Sensor dadurch gekennzeichnet, dass geometrisch um die Haupt-Sensorelemente herum mehrere Hilfs-Sensoren, z.B. drei (7) (8) (9), angeordnet sind. Die Hilfs-Sensoren definieren die Kontaktfläche (5), innerhalb der die Haupt-Sensoren zu liegen kommen. Wird nun detektiert, dass alle Hilfs-Sensoren kontaktiert werden, ist gewährleistet, dass die geometrisch innerhalb der Kontaktfläche liegenden Haupt-Sensorelemente (1) (2) (3) abgedeckt und vollständig abgedeckt von der Haut berührt werden. Dabei müssen die Hilfs-Sensoren nicht vollständig abgedeckt sein, sie müssen nur ungefähre Messdaten liefern wie Kontakt oder nicht Kontakt.

Die durch die Hilfs-Sensoren aufgespannte Fläche und die Haupt-Sensorelemente müssen sich nicht vollständig überlagern. Es muss nur konstruktiv gewährleistet sein, dass bei Abdeckung der Hilfs-Sensoren die Kontaktfläche für die Haupt-Sensoren so groß ist, dass die Hauptsensoren innerhalb liegen und damit vollständig abgedeckt sind. Z.B. beim Daumen ist mit dem Aufspannen einer Dreiecks-Fläche durch die Hilfs-Sensoren durchaus gewährleistet, dass die auch etwas über die Dreiecksfläche hinausragenden Haupt-Sensorelemente korrekt abgedeckt sind. Dies deshalb, weil das Auflagemuster des Daumens auf einem Sensor, der an der Seite einer Computermaus montiert ist, oval ist. Wenn alle 3 Hilfssensoren bedeckt sind, kann daraus geschlossen werden, dass auch die etwas über die Dreiecksfläche ragenden Haupt-Sensorelemente vollständig abgedeckt sind.

Die Unterscheidung zwischen den Signalen der Haupt-Sensorelemente und den Signalen der Hilfs-Sensorelemente muss gewährleistet sein, siehe Figur 2 "Beispiel eines Signalverlaufes von Hilfssensorelementen und Hauptsensorelementen für einen Daumensensor einer Computermaus". Im Falle dieses Beispieles für die Messung des Hautwiderstandes am Daumensensor (13) nach Figur 1 könnte dies durch die Verwendung einer Auswertung mittels Wechselstrom (10) für die Hilfs-Sensorelemente (7), (8), (9) und einer Auswertung mittels Gleichstrom (11) für die Haupt-Sensorelemente (1), (2) geschehen. Diese Signale sind voneinander relativ leicht zu trennen. Hilfssensorelemente und Hauptsensorelemente haben jeweils einen Messteil, deren Ausgangssignale an das Datenverarbeitungssystem (12) zur Auswertung geliefert werden. Diese Auswertung beinhaltet die Abdeckdetektion sowie die Messung mit vollständig abgedeckten Hauptsensoren.

Alternativ können die Messungen mit den Hilfssensoren zur Detektion des Sensorelementkontaktes und die Haupt-Messung mit der gleichen Messgröße zeitlich hintereinander durchgeführt werden. Dabei wird angenommen dass während den Hauptmessungen (zwischen den Detektionsmessungen) der Sensorkontakt mit der Messstelle unverändert bleibt.

### 1.4.1.1.2. Beispiel Fingersensor

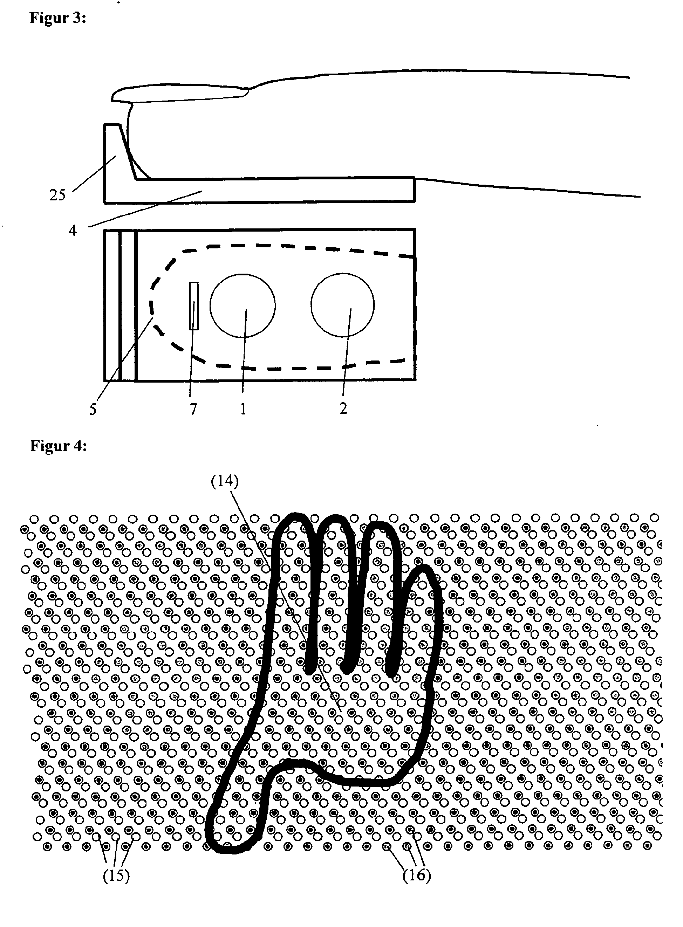

Als weiteres Beispiel sei ein Fingersensor für ein physiologisches Messsystemes nach Figur 3 "Schematische Ansicht und Aufsicht eines Fingersensors mit gemeinsamer Referenzelektrode für Haupt- und Nebensensoren" angeführt um zu erläutern, dass die Haupt- und Hilfssensoren gemeinsame Elektroden haben können und dass durch die geometrische Anordnung dennoch die vollständige Abdeckung für die Hauptmessung gewährleistet sein kann.

Der Sensor (4) mit einem Auflagehindernis (25) wird and die Haut einer Fingerbeere eines Fingers mit seiner Kontaktfläche (5) angelegt, das Auflagemuster der Kontaktfläche dieses Sensors ist länglich.

Wird nun zwischen dem Hilfssensorelement (7) und der Kontaktfläche (2) durch eine Messung Kontakt festgestellt, ist gewährleistet dass das Hauptsensorelement (1) vollständig bedeckt ist. Durch das Wissen um die Lage der Kontaktflächen und die konstruktive Ausbildung des Sensors, der wegen dem Auflagehindernis nur an eine Fingerbeere angelegt werden kann ist gewährleistet, dass das Sensorelement (2), das in diesem Fall sowohl als Haupt- wie auch als Hilfssensorelement dient, vollständig bedeckt ist und damit eine korrekte Messung zwischen den Sensorelementen (1) und (2) durchgeführt werden kann.

Die Unterscheidung zwischen den Signalen der Haupt-Sensorelemente und den Signalen der Hilfs-Sensorelemente kann z.B. wieder durch die Verwendung von Wechselstrom für die Hilfs-Sensorelemente und von Gleichstrom für die Haupt-Sensorelemente geschehen.

Alternativ können die Messungen mit dem Hilfssensor zur Detektion des Sensorelementkontaktes und die Haupt-Messung mit der gleichen Messgröße (z.B. Gleichstrom) zeitlich hintereinander durchgeführt werden. Dabei wird angenommen dass während den Hauptmessungen (zwischen den Detektionsmessungen) der Sensorkontakt mit der Messstelle unverändert bleibt.

### 1.4.1.1.3. Beispiel Lenkrad

Als weiteres Beispiel sei als Sensor ein Lenkrad mit einer Vielzahl von Sensorelementen zur Messung des Hautwiderstandes angeführt. In Figur 4 "Schematische Ansicht der Sensorik zurErmittlung der Kontaktfläche am Lenkrad" ist die zu berührende Fläche des Lenkrades abgerollt schematisch als Rechteck dargestellt. Um zu ermitteln, welche Haupt-Sensorelemente (15, weiß) von der Messstelle Haut (der berührenden Hand) abgedeckt sind und so ihre Funktion möglichst fehlerfrei erfüllen können, sind zwischen den Haupt-Sensorelementen die Hilfs-Sensoren (16, grau) angeordnet. Wird nun detektiert, welche der Hilfs-Sensoren von der Haut berührt werden, ergibt dies für das Messsystem die Kontaktfläche (14). Alle innerhalb dieser Kontaktfläche liegenden Haupt-Sensoren sind nun vollständig abgedeckt und werden für die aktuelle Messung herangezogen. Dabei müssen die Hilfs-Sensoren nicht vollständig abgedeckt sein, sie müssen nur ungefähre Messdaten liefern wie Kontakt oder nicht Kontakt.

Die Unterscheidung zwischen den Signalen der Haupt-Sensorelemente und den Signalen der Hilfs-Sensorelemente kann z.B. wieder durch die Verwendung von Wechselstrom für die Hilfs-Sensorelemente und von Gleichstrom für die Haupt-Sensorelemente geschehen oder auch mit der gleichen Messgröße (z.B. Gleichstrom) zeitlich hintereinander durchgeführt werden wobei angenommen wird, dass während den Hauptmessungen (zwischen den Detektionsmessungen) der Sensorkontakt mit der Messstelle unverändert bleibt.

### 1.4.1.2. Mittels Abdeck-Detektion der Sensorelemente zueinander

Hier sind die Sensorelemente nicht in Hauptsensoren und Hilfssensoren getrennt. Die Erfindung ist durch eine Vielzahl von Sensoren gekennzeichnet, die auf der zu berührende Sensorfläche angebracht sind. Die geometrische Lage der Sensorelemente zueinander ist dem Messsystem bekannt.

Um zu ermitteln, welche Sensorelemente abgedeckt sind und so ihre Funktion möglichst fehlerfrei erfüllen können, ist diese Messvorrichtung dadurch gekennzeichnet, dass jedes Sensorelement in Matrixform abgetastet wird um mit einem dazwischen fließendem Hilfs-Medium wie Strom, Licht o.ä. oder einer anderen Eigenschaft wie z.B. der Temperatur zu detektieren, ob und wo ein Kontakt mit der Messstelle stattfindet. Dadurch ergibt sich die Kontaktfläche der abgedeckten Sensorelemente. Alle innerhalb dieser Kontaktfläche liegenden Sensorelemente sind nun vollständig abgedeckt und werden für die aktuelle Messung herangezogen. Auf Sensorelemente im Randbereich der Kontaktfläche ist Bedacht zu nehmen, die Messwerte sind eventuell zu verwerfen oder, falls möglich, zu korrigieren.

Die Information der Lage der Kontaktfläche selbst kann ebenfalls zur weiteren Verarbeitung herangezogen werden.

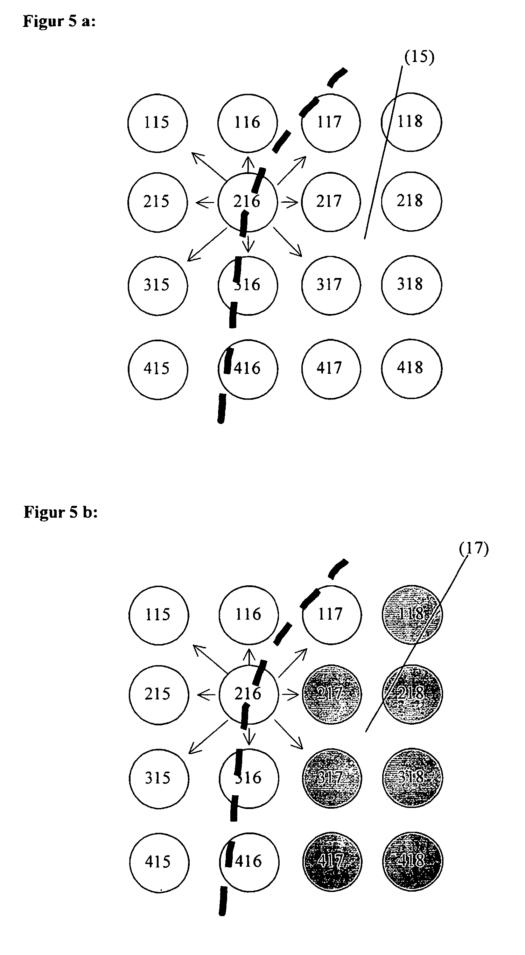

Als Beispiel sei wieder ein Lenkrad genannt und auf Figur 5 a "Schematische Ansicht der Anordnung zur Ermittlung der Kontaktfläche durch Messung mit den Sensorelemente zueinander" verwiesen wo ein kleiner Teil der zu berührende Fläche abgerollt vom Lenkrad als Rechteck darstellt ist und die von der Haut berührte Kontaktfläche (15) ermittelt werden soll. Die Sensorelemente sind in einer Matrix angeordnet, im Beispiel 100 Sensoren je Zeile, in der Zeichnung befindet sich jeweils die Nummer des Sensorelementes im Inneren des Sensorelementes. Nun wird mit einem Sensorelement nach dem anderen, hier z.B, Nr. 216, und seinen geometrisch umliegenden Elemente 115, 116, 117, 215, 217, 315, 316 und 317 je eine Messung durchgeführt, abgetastet. Die Auswertung der Messignale ergibt im angenommenen Beispiel Kontakt über die Messstelle mit den Elementen 117, 217, 316 und 317.

Mit diesen Informationen kann das System feststellen, dass die geometrische Grenze der Messstelle über den Elementen 117, 216, 316 und (nach einer Messung mit dem Element 316 und seinen umliegenden Elementen) 416 verläuft.

Die festgestellte Kontaktfläche (17, grau dargestellte Sensorelemente) in der Figur 5 b "Schewatische Ansicht der Kontaktfläche nach der Abdeck-Detektion mittels Messung der Sensorelemente zueinander" mit vollständig abgedeckten Sensoren ist demnach rechts ab den grau dargestellten Sensoren 118, 217, 218 , 317, 378, 417, 418 detektiert. Nach Durchführung aller Messungen von allen Sensorelementen mit ihren jeweils umliegenden Sensorelementen kann die vollständige Kontaktfläche ermittelt werden. Mit dieser Information wird die eigentliche möglichst fehlerfreie Hauptmessung durchgeführt während der angenommen wird, dass sich die Kontaktfläche nicht verändert, sowie, falls erforderlich die Information der Lage der Kontaktfläche weiterverarbeitet.

Als Variante der Abtastung kann für jedes Sensorelement eine Messung mit einer oder mehreren gemeinsamen Referenzelektroden durchgeführt werden. Als Referenzelektroden könnte jedes beliebige abgedeckte Sensorelement verwendet werden. Diese Referenzelektroden können sogar erst während der Messung bestimmt werden, wenn in einem ersten Messdurchgang eine oder mehrere bedeckte Sensorelemente ermittelt werden, die für die nächsten Messdurchgänge als Referenzelektroden wirken.

### 1.4.2. Nachführung und/oder Anpassen der Sensorelemente

Die Sensorelemente müssen an der interessierenden Messstelle gut anliegen, der Kontakt muss gewährleistet sein (Sensor stationär auf einer Messstelle oder auch sich auf der Messstelle bewegend). Im Falle von erstem Anlegen und Bewegung während der Messung wird dies erfindungsgemäß durch ein Nachführen und/oder ein Anpassen der Sensorelemente gelöst.

Daher ist die Erfindung dadurch gekennzeichnet, dass die Sensorelemente entsprechend den zu erwartenden Bewegungen so weit wie möglich nachgeführt werden um bei Bewegung der Messstelle den Kontakt mit ihr nicht zu verlieren und so Messfehler zu vermeiden.

Zusätzlich oder alternativ ist die Erfindung dadurch gekennzeichnet, dass die Sensorelemente selbst an die Oberflächenform der Messstelle angepasst werden können, um einen korrekten Kontakt zu gewährleistet. Dabei sind die Sensorelemente formbar und/oder beweglich und werden so an die Messstelle, auch bei Bewegungen, angepasst.

Dies kann mit und ohne zu Hilfenahme einer starren Hilfssensorenmatrix durchgeführt werden. Dabei sind die Hilfssensoren nicht beweglich ausgeführt und dienen als Widerlager um die mechanischen Kräfte der Messstelle aufzunehmen.

### 1.4.2.1. Beispiel Wippe für Daumensensor einer Computermaus

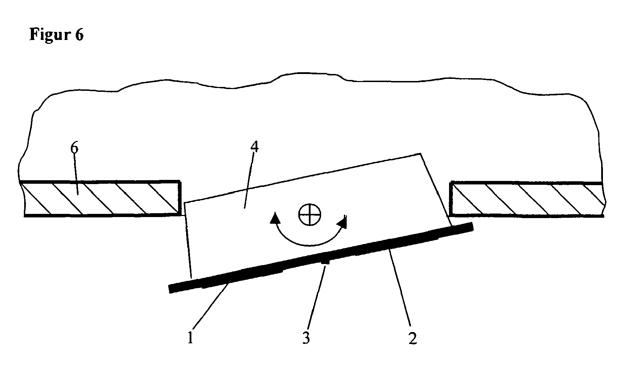

In Figur 6 "Aufsicht auf bewegliches Element mit Sensorelementen zur Nachführung" sind die Sensorelemente (1), (2) für den Hautwiderstand und (3) für die Temperatur auf der vertikalen Fläche eines beweglichen Elementes (4) (eine Wippe) eingelassen. Diese Wippe ist im Bereich des Daumens in die Maus (6) eingefügt und folgt dem Daumen bei der Bewegung bei der Arbeit mit der PC-Maus. Durch dieses Nachführen wird in weiten Grenzen der Kontakt zwischen Sensorelementen und Haut sichergestellt.

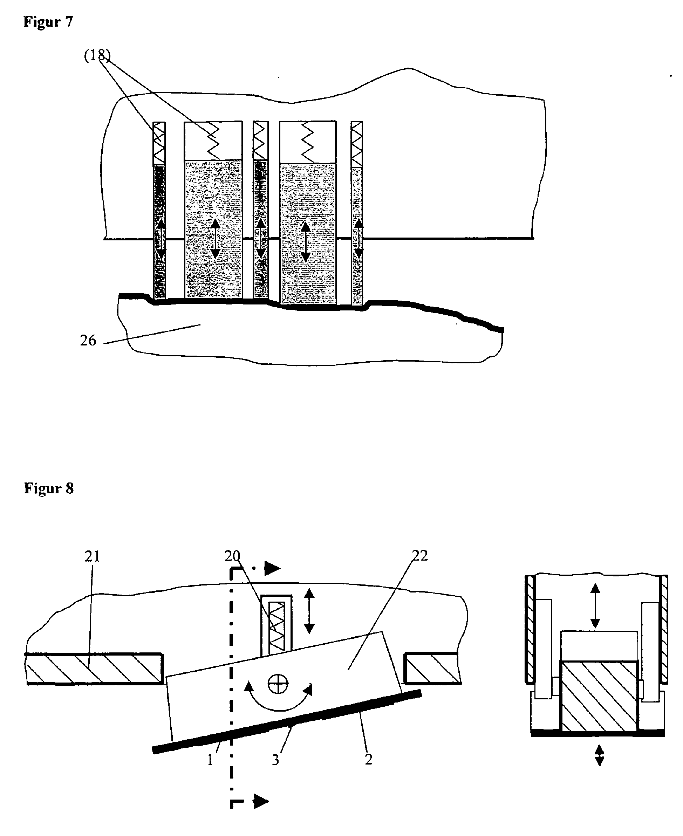

Alternativ oder zusätzlich können auch die Sensorelemente (1), (2), (3) an die Oberflächenform der Haut angepasst werden, um einen korrekten Kontakt zur Haut zu gewährleisten, siehe Figur 7 "Aufsicht auf bewegliche Sensorelemente zur Anpassung an die Messstelle". Dabei sind die Sensorelemente beweglich mit Federn (18) versehen und passen sich so an die Haut (26) an auch bei Bewegungen.

Alternativ oder zusätzlich können auch die Sensorelemente (1), (2), (3) selbst an die Oberflächenform der Haut angepasst werden, um einen korrekten Kontakt zur Haut zu gewährleisten. Dabei sind die Sensorelemente selbst formbar, nehmen unter leichtem Druck die Form der Hautstelle an und passen sich so an die Haut auch bei Bewegungen an. Dazu muss das Material der Sensorelemente formbar sein.

### 1.4.2.2. Beispiele der Anwendung an Joysticks, Spielekonsolen, mobilen Datenverarbeitungsgeräten und mobilen Telefonen

Ein Joystick ist ein Computereingabegerät, das wie ein Flugzeugsteuerknüppel bedient wird, eine Spielekonsole wird meist zur Kontrolle von elektronischen Spielen mittels Eingabeknöpfen verwendet.

Laut Figur 8 "Aufsicht auf bewegliches, gefedertes Element mit Sensorelementen zur Nachführung sowie im Falle des Hautleitwertes zur Konstanthaltung des Druckes" sind in diesem Beispiel Sensorelemente in einem beweglichen Element eingelassen. Dies ist hier eine Wippe (22) mit gleichzeitig mit der Feder (20) gefederter hinein drückbarer Vertikalführung. Dieser Sensor ist z.B. im Bereich des Handballens in den Joystick (21) oder die Spielkonsole oder im Bereich der Handfläche in das mobile Datenverarbeitungsgerät oder das mobile Telefon eingefügt und folgt der Haut bei den Bewegungen bei der Verwendung.

Alternativ oder zusätzlich können auch die Sensorelemente (1), (2), (3) selbst an die Oberflächenform der Haut angepasst werden, um einen korrekten Kontakt zur Haut zu gewährleisten, ähnlich wie in Figur 7 für die Computermaus. Dabei sind die Sensorelemente formbar und/oder federnd beweglich und werden so an die Haut, auch bei Bewegungen, angepasst.

Bei nicht so strengen Forderungen an die Messgenauigkeit kann auf die Nachführung verrichtet werden, die Sensorelemente werden dann mechanisch nicht beweglich eingebaut (z.B. im Handtellerbereich).

### 1.4.3. Druckkonstanthaltung für die Hautwiderstandsmessung

Ein Faktor bei der Messung des elektrischen Hautwiderstandes ist der Druck, den die Kontaktflächen auf die Haut ausüben. Ändert sich der Druck während der Messung durch mehr oder weniger festes Zugreifen des Anwenders, können Änderungen im Wert der elektrischen Hilfsgröße zur Ermittlung des Hautwiderstandes auftreten, die nicht von der Hautleitfähigkeit resultieren (Messfehler). Daher ist die Erfindung dadurch gekennzeichnet, daß die Kontaktflächen für den Hautwiderstand federnd angeordnet sind. Druckänderungen der Hautstelle werden nur auf das die gefederten Kontaktflächen umliegende Material übertragen. Der Auflagedruck der Kontaktflächen auf die Haut hängt damit in weiten Bereichen nur mehr von der Federung ab, die einen konstanten Auflagedruck erzeugt.

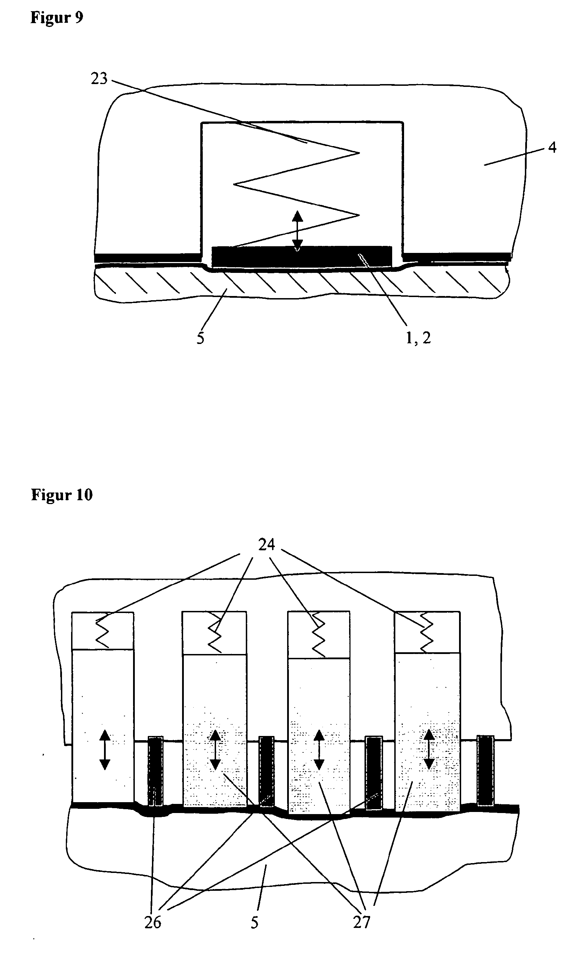

In Figur 9 "Federnde Kontaktflächen für die Hautwiderstandsmessung " werden die in den Sensor (4) montierten gefederten Sensorelemente (1) oder (2) dargestellt, auf die die Haut (5) drückt. Durch die Feder (23) wird der konstante Auflagedruck der Hauptsensoren erzeugt, die nun möglichst fehlerfrei messen können. Druckänderungen werden nur auf das umliegende Sensormaterial (4) übertragen.

Im Beispiel nach Figur 10 "Federnde Kontaktflächen für die Messwerterfassung in starrer Hilfssensorenmatrix zur Konstanthaltung des Auflagedruckes bei Messung des Hautleitwertes" kann dies auch mittels einer starren Hilfssensorenmatrix durchgeführt werde. Hier wird die Haut (5) von starten Hilfssensoren (26, dunkelgrau) positioniert, auf die die Druckänderungen übertragen werden. Für deren Messsignale spielt ein konstanter Auflagedruck keine große Rolle solange sie überhaupt kontaktiert werden. Die (hellgrauen) Hauptsensoren (27) werden mit den Federn (24) gegen die Haut gedruckt. Durch die Positionierung der Haut (5) durch Auflage an die Hilfssensoren ergibt sich ein konstanter Auflagedruck der Hauptsensoren, die nun mit konstantem Auflagedruck möglichst fehlerfrei messen können.

Alternativ dazu ist die Erfindung dadurch gekennzeichnet, dass die Federung des gesamten Sensorelementes zur Druckkonstanthaltung herangezogen wird. Wenn sich die Hautstelle nur innerhalb eines definierten Bereiches aufhalten kann, ist es möglich, durch Nachführung des gesamten Sensorelementes den Auflagedruck in Grenzen konstant zu halten und damit die Messfehler klein zu halten.

Ein Beispiel wäre ein Joystick dessen gefedertes Element nach Figur 8 solange mit einigermaßen gleichbleibendem Druck die Haut erreicht, als die Hand den Joystickknüppel umschließt

### 1.5. Struktur der Auswertesoftware

Dies beschreibt die Struktur von Software in einer Datenverarbeitungseinheit, die die Messdaten der fehlervermindernden Messsensorik verarbeitet.

### 1.5.1. Abdeckdetektion mit Hilfssensorelementen

Die Auswertesoftware für die artefaktvermindernden Sensoren ist gekennzeichnet dadurch, dass durch die Abdeckdetektion nach Kapitel 1.4.1.1 die Kontaktfläche ermittelt werden kann. Mit der dem System bekannten Anordnung der Sensorelemente wird damit unterschieden zwischen den Zuständen "Keines der Haupt- und Hilfs-Sensorelemente abgedeckt", "bestimmte Haupt- und Hilfs-Sensorelemente abgedeckt, aber nicht vollständig" und "bestimmte Haupt- und Hilfs-Sensorelemente vollständig abgedeckt".

Bei den Zuständen "Keines der Haupt- und Hilfs-Sensorelemente abgedeckt" und "bestimmte Haupt- und Hilfs-Sensorelemente abgedeckt, aber nicht vollständig" werden die Messwerte verworfen oder falls möglich korrigiert und damit Messfehler eliminiert, weiters kann an den Anwender eine Warnung ausgegeben werden und/oder eine Aktion zur Sensorenkorrektur eingefordert werden.

Beim Zustand "bestimmte Haupt- und Hilfs-Sensorelemente vollständig abgedeckt" können die ermittelten abgedeckten Sensorelemente für möglichst fehlerfreie Messungen herangezogen werden.

### 1.5.2. Abdeck-Detektion der Sensorelemente zueinander

Die Auswertesoftware für die artefaktvermindemden Sensoren mit der Abdeckdetektion der Sensorelemente zueinander ist gekennzeichnet dadurch, dass durch die Abdeckdetektion nach Kapitel 1.4.1.2 die Kontaktfläche ermittelt werden kann. Mit der dem System bekannten Anordnung der Sensorelemente wird damit unterschieden zwischen den Zuständen "Keine Sensorelemente abgedeckt" und "bestimmte Sensorelemente abgedeckt, und damit vollständig abgedeckt".

Beim Zustand "Keine Sensorelemente abgedeckt" werden die Messwerte verworfen oder falls möglich korrigiert und damit Messfehler eliminiert, weiters kann an den Anwender eine Warnung ausgegeben werden und/oder eine Aktion zur Sensorenkorrektur eingefordert werden.

Beim Zustand "bestimmte Sensorelemente abgedeckt und damit vollständig abgedeckt" können die ermittelten abgedeckten Sensorelemente für möglichst fehlerfreie Messungen herangezogen werden.

## Patentansprüche

1. Messvorrichtung zum Messen von physiologischen Größen von einer menschlichen oder tierischen Hautoberfläche und zum Ermitteln von ungenauem Anlegen von Messstellen auf Messsensoren und zur Anwendung in robusten Meßsystemen, die Werte unter realistischen und schwierigen Bedingungen messen; und die Meßvorrichtung besteht aus:
einer Mehrzahl von Sensorelementen zur Durchführung von physiologischen Messungen, wobei die Sensorelemente auf einer Sensorfläche befestigt sind und in eine Anzahl von Hauptsensoren (1, 2, 3) und Hilfssensoren (7, 8, 9) aufgeteilt sind; und
einer Daten-Verarbeitungs-Einheit (12) die ausgelegt ist zum Unterscheiden zwischen Signalen von den Hauptsensoren (1, 2, 3) und den Hilfssensoren (7, 8, 9) zur Ermittlung von ungenauem Anlegen von Messstellen auf Messsensoren;
wobei die relativen Positionen der Haupt- und Hilfssensoren zueinander der Daten-Verarbeitungs-Einheit (12) bekannt sind, so daß durch Signale der Hauptsensoren und Hilfssensoren eine Kontaktfläche (5) ermittelt wird, innerhalb der alle Hauptsensoren vollständig abgedeckt sind; und die Daten-Verarbeitungs-Einheit (12) ist so ausgelegt daß sie nur Signale von vollständig bedeckten Hauptsensoren (1, 2, 3) verwendet und, falls erwünscht, die signale von vollständig bedeckte Hilfssensoren (7, 8, 9) innerhalb der ermittelten Kontaktfläche (5) für tatsächliche Messungen, verwendet

2. Die Messvorrichtung nach Anspruch 1, wobei die physiologischen Größen den Hautwiderstand beinhalten.

3. Die Messvorrichtung nach Anspruch 1 oder 2, wobei besagte Messvorrichtung zumindest einen Referenzsensor beinhaltet.

4. Die Messvorrichtung nach einem der vorstehenden Ansprüchen, wobei die Anzahl der Sensorelemente auf der Sensorfläche in Matrixanordnung ausgelegt ist, und die Sensorelementen gleichzeitig oder abwechselnd als Hauptsensoren (15), Hiffssensoren (16) und, falls erforderlich, als Referenzsensoren dienen; und die Sensorelemente sind ausgelegt zum Messen nach Matrix-Art um so eine Kontaktfläche (14) des Testgebietes zu ermitteln welches sich nur aus vollständig bedeckten Sensorelementen zusammensetzt.

5. Die Messvorrichtung nach einem der vorstehenden Ansprüchen, wobei die Anzahl der Sensorelemente beweglich ist so daß Kontakt mit dem Testgebiet bei erstem Kontakt oder während Bewegung nachgeführt und aufrechterhalten werden kann, womit Meßfehler vermieden werden.

6. Die Messvorrichtung nach einem der vorstehenden Ansprüchen, wobei die Hauptsensorelemente und die Hilfssensorelemente formbar sind.

7. Die Messvorrichtung nach einem der vorstehenden Ansprüchen, wobei die Anzahl der Sensorelemente federnd ist so daß Kontakt mit dem Testgebiet bei erstem Kontakt oder während Bewegung nachgeführt und aufrechterhalten werden kann, womit Meßfehler vermieden werden.

8. Die Messvorrichtung nach einem der vorstehenden Ansprüchen, wobei die Anzahl der Sensorelemente oder die Meßvorrichtung selbst federnd gelagert ist/sind um einen so konstant wie möglichen Lagedruck herzustellen um somit Meßfehler zu verhindern die bedingt sind durch variablen Lagedruck der Haut auf den Sensorelementen.

9. Die Messvorrichtung nach einem der vorstehenden Ansprüchen, wobei die Datenverarbeitungseinheit zwischen den Zuständen "keine Sensorelemente abgedeckt" und "bestimmte Sensorelemente vollständig abgedeckt" unterscheiden kann.

10. Die Messvorrichtung nach Anspruch 9, wobei die Datenverarbeitungseinheit eine Warnung erzeugt,wenn der Zustand "bestimmte Sensorelemente vollständing abgedeckt" nicht erreicht wird.

11. Die Messvorrichtung nach einem der vorstehenden Ansprüchen, wobei die Datenverarbeitungseinheit die Lage der Kontaktfläche analysieren kann.

## Claims

1. Measuring device to measure physiological values from a human or animal skin surface and to detect non-precise application of testing sites onto measuring sensors and for use in robust measuring systems which measure values under realistic and challenging conditions; and the measuring device is comprised of
a plurality of sensor elements for the performance of physiological measurements whereby the sensor elements are disposed on a sensor surface and are divided into a number of main sensors (1, 2, 3) and auxiliary sensors (7, 8, 9); and
a data processing unit (12) which is designed to differentiate between signals from the main sensors (1, 2, 3) and the auxiliary sensors (7, 8, 9) to detect non-precise application of testing sites onto measuring sensors;
whereby the relative positions of the main sensors and the auxiliary sensors are known to the data processing unit (12), so that through signals from the main sensors and the auxiliary sensors a contact area (5) is determined within which all main sensors are completely covered; and the data processing unit (12) is so designed that it only uses signals from completely covered main sensors (1, 2, 3) and, if so desired, uses the signals of completely covered auxiliary sensors (7, 8, 9) within the determined contact area (5) for actual measurements.

2. The measuring device of claim 1
wherein the physiological values include the skin resistance.

3. The measuring device of claim 1 or 2
wherein said measuring device contains at least one reference sensor.

4. The measuring device according to one of the aforesaid claims,
whereby the plurality of sensor elements on the sensor surface is arranged in the form of a matrix, and the sensor elements can serve at the same time or consecutively as main sensors (15), auxiliary sensors (16) and, if necessary, as reference sensors; and the sensor elements are designed to measure in matrix fashion to determine a contact area (14) of the test area which consists only of completely covered sensor elements.

5. The measuring device according to one of the aforesaid claims,
whereby the plurality of sensor elements is moveable so that the contact with the test area at first contact or during movements can be tracked and sustained, whereby measuring errors are avoided.

6. The measuring device according to one of the aforesaid claims,
whereby the main sensor elements and the auxiliary sensor elements are pliable.

7. The measuring device according to one of the aforesaid claims,
whereby the plurality of sensor elements is spring mounted so that the contact with the test area at first contact or during movements can be tracked and sustained, whereby measuring errors are avoided.

8. The measuring device according to one of the aforesaid claims,
whereby the plurality of sensor elements or the measuring device itself is/are spring mounted in order to produce a constant as possible bearing pressure to thereby avoid measurement errors caused by variable bearing pressure of the skin onto the sensor elements.

9. The measuring device according to one of the aforesaid claims,
whereby the data processing unit can discern between the states "no sensor elements covered" and "certain sensor elements completely covered".

10. The measuring device according to claim 9,
whereby the data processing unit creates a warning if the state "certain sensor elements completely covered" is not attained.

11. The measuring device according to one of the aforesaid claims,
whereby the data processing unit can analyze the geometric position of the contact area.

## Revendications

1. Dispositif de mesure pour mesurer des grandeurs physiologiques d'une surface de peau d'homme ou d'animal et pour déterminer une application imprécise d'emplacements de mesure sur des capteurs de mesure et pour l'application dans des systèmes de mesure robustes, qui mesurent des valeurs sous des conditions réalistes et difficiles; et le dispositif de mesure est constitué:
d'une pluralité d'éléments de capteur pour l'exécution de mesures physiologiques, où les éléments de capteur sont fixés sur une face de capteur et sont répartis en un nombre de capteurs principaux (1, 2, 3) et de capteurs auxiliaires (7, 8, 9); et
d'une unité de traitement de données (12) qui est conçue pour distinguer entre des signaux des capteurs principaux (1, 2, 3) et des capteurs auxiliaires (7, 8, 9) pour la détermination d'une application imprécise d'emplacements de mesure sur des capteurs de mesure;
où les positions relatives des capteurs principaux et auxiliaires entre eux sont connues par l'unité de traitement de données (12) de sorte que par des signaux des capteurs principaux et des capteurs auxiliaires, une face de contact (5) est déterminée, à l'intérieur de laquelle tous les capteurs principaux sont entièrement recouverts; et l'unité de traitement de données (12) est conçue de façon à utiliser seulement des signaux de capteurs principaux (1, 2, 3) entièrement couverts et, si cela est souhaité, utilise les signaux de capteurs auxiliaires (7, 8, 9) entièrement recouverts à l'intérieur de la face de contact déterminée (5) pour des mesures effectives.

2. Dispositif de mesure selon la revendication 1, où les grandeurs physiologiques comprennent la résistance de la peau.

3. Dispositif de mesure selon la revendication 1 ou 2, où le dispositif de mesure précité comprend au moins un capteur de référence.

4. Dispositif de mesure selon l'une des revendications précédentes, où le nombre d'éléments de capteur sur la face de capteur est disposé selon un agencement de matrice, et les éléments de capteur servent en même temps ou alternativement de capteurs principaux (15), de capteurs auxiliaires (16) et, en cas de besoin, de capteurs de référence; et les éléments de capteur sont conçus pour la mesure d'un type de matrice pour déterminer ainsi une face de contact (14) de la zone de test qui est constituée seulement d'éléments de capteur entièrement recouverts.

5. Dispositif de mesure selon l'une des revendications précédentes, où le nombre d'éléments de capteur est mobile de sorte que le contact avec la zone de test, lors d'un premier contact ou pendant un mouvement, peut être suivi et maintenu, moyennant quoi des erreurs de mesure sont évitées.

6. Dispositif de mesure selon l'une des revendications précédentes, où les éléments des capteurs principaux et les éléments des capteurs auxiliaires peuvent être formés.

7. Dispositif de mesure selon l'une des revendications précédentes, où le nombre des éléments de capteur est élastique de sorte que le contact avec la zone de test, lors d'un premier contact ou pendant le mouvement, peut être suivi et maintenu, moyennant quoi des erreurs de mesure sont évitées.

8. Dispositif de mesure selon l'une des revendications précédentes, où le nombre des éléments de capteur ou bien le dispositif de mesure lui-même est/sont logés eux-mêmes à ressort pour établir une pression de position aussi constante que possible pour éviter ainsi des erreurs de mesure qui proviennent d'une pression de position variable de la peau sur les éléments de capteur.

9. Dispositif de mesure selon l'une des revendications précédentes, où l'unité de traitement de données peut distinguer entre les états "pas d'éléments de capteur recouverts" et "certains éléments de capteur entièrement recouverts".

10. Dispositif de mesure selon la revendication 9, où l'unité de traitement de données émet un avertissement lorsque l'état "certains éléments de capteur entièrement recouverts" n'est pas atteint.

11. Dispositif de mesure selon l'une des revendications précédentes, où l'unité de traitement de données peut analyser la position de la face de contact.
